(19) Europäisches Patentamt / European Patent Office / Office européen des brevets

(11) **EP 4 181 992 B1**

(12) **EUROPÄISCHE PATENTSCHRIFT**

(45) Veröffentlichungstag und Bekanntmachung des Hinweises auf die Patenterteilung:
**11.06.2025 Patentblatt 2025/24**

(21) Anmeldenummer: **21752502.1**

(22) Anmeldetag: **19.07.2021**

(51) Internationale Patentklassifikation (IPC):
**A61M 16/00** *(2006.01)* **A61M 16/20** *(2006.01)*
**A61M 16/10** *(2006.01)* **A61M 16/16** *(2006.01)*

(52) Gemeinsame Patentklassifikation (CPC):
**A61M 16/0066; A61M 16/024;** A61M 16/0051;
A61M 16/0069; A61M 16/0087; A61M 16/1065;
A61M 16/107; A61M 16/1075; A61M 16/16;
A61M 16/208; A61M 2016/0027; A61M 2016/0036;
A61M 2205/3673; A61M 2230/205

(86) Internationale Anmeldenummer:
**PCT/EP2021/070158**

(87) Internationale Veröffentlichungsnummer:
**WO 2022/018031 (27.01.2022 Gazette 2022/04)**

(54) **BEATMUNGSVORRICHTUNG ZUR BEATMUNG EINER PERSON**

VENTILATION APPARATUS FOR VENTILATING A PERSON

DISPOSITIF RESPIRATOIRE POUR VENTILER UN INDIVIDU

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.07.2020 DE 102020119085**

(43) Veröffentlichungstag der Anmeldung:
**24.05.2023 Patentblatt 2023/21**

(73) Patentinhaber: **Max-Planck-Gesellschaft zur Förderung der Wissenschaften e.V. 80539 München (DE)**

(72) Erfinder: **HELLEIS, Frank 55124 Mainz (DE)**

(74) Vertreter: **v. Bezold & Partner Patentanwälte - PartG mbB Ridlerstraße 57 80339 München (DE)**

(56) Entgegenhaltungen:
WO-A1-2018/204985    JP-A- 2018 531 716
US-A1- 2018 064 894    US-A1- 2019 269 871

**Beschreibung**

Die Erfindung betrifft eine Beatmungsvorrichtung, die zur künstlichen, nicht-invasiven (NIV-) Beatmung einer Person eingerichtet ist, und insbesondere ein Radialgebläse enthält.

In der Beatmungstechnik sind verschiedene Typen von Beatmungsgeräten bekannt, die sich in Bezug auf ihre Konfigurationen und Betriebsweisen unterscheiden (siehe z. B. DE 10 2008 047 026 B4, WO 2009/112 076 A1, DE 10 2008 005 558 A1 und DE 10 2018 003 027 A1). Der Beatmungsdruck eines Beatmungsgases kann in einem Beatmungsgerät z. B. durch die Verbindung mit einem Druckgasreservoir (siehe z. B. C. Galbiati et al., 2020, arXiv:2003.10405) oder durch ein Gebläse erzeugt werden. Als Gebläse kann ein Radialgebläse verwendet werden (siehe z. B. DE 200 16 769 U1), das aufgrund seines kompakten Aufbaus Vorteile hat. Relevante Dokumente aus dem Stand der Technik sind ferner: US 2019/269871 A1, US 2018/064894 A1, JP 2018 531716 A und WO 2018/204985 A1.

Typischerweise wird bei modernen Beatmungsgeräten eine BIPAP-Beatmung (engl.: Biphasic Positive Airway Pressure) realisiert, also ein druckkontrolliertes Verfahren, bei dem mit zwei unterschiedlichen Druck-Niveaus beatmet wird, die jeweils bei der Einatmung (Inspiration) oder der Ausatmung (Exspiration) eingestellt werden. Gegenüber den älteren volumenkontrollierten haben druckkontrollierte Verfahren prinzipbedingt ohne zusätzlichen Aufwand den zentralen Vorteil, den Patienten in seinen Spontanatmungsbemühungen während der Entwöhnungsphase zu unterstützen. Wichtige Beatmungsparameter sind damit insbesondere der positive endexspiratorische Druck (englisch: positive end-expiratory pressure, PEEP), der den positiven Druck in der Lunge am Ende der Ausatmung (Exspiration) bezeichnet, und der maximale Druck, der bei der Einatmung (Inspiration) auftritt (Spitzendruck oder Ppeak oder PIP).

Bei vielen herkömmlichen Beatmungsgeräten werden diese Druckniveaus durch (Proportional-) Ventile eingestellt, die aber prinzipbedingt nicht den Druck selbst kontrollieren, sondern den Fluss, der erst im Zusammenspiel mit dem Lungenvolumen des Patienten zum gewünschten Druck führt. Bei Ein-Schlauch-Systemen z.B. kann die Ausatemluft zur Minimierung der $CO_2$-Rückatmung nahe der Atemmaske durch ein kontrolliertes Leck, ein passives differenzdruck-gesteuertes Ventil oder ein aktives Proportionalventil entfernt werden. Bei Zwei-Schlauch-Systemen können aktive Komponenten mit dem zusätzlichen Nachteil noch höherer Kosten in das Steuergerät integriert werden.

Von Nachteil sind jedoch die äußerst hohe apparative (Ventile, Schläuche, zusätzliche Filter zum Schutz der Umgebung), sensorische (doppelte Flussmessung) und algorithmische Komplexität (Sicherheitseinrichtungen bei Stromausfall oder Fehlfunktion) und damit erheblich höhere Kosten oder fehlende wichtige diagnostische Optionen, wie das Ausatemvolumen oder die Bestimmung eines Maskenlecks. Wenn z. B. Maskenlecks online diagnostizierbar und klein genug wären, könnte die Atemluft durch einfache HME (Heat-Moisture-Exchange) Filter temperiert und befeuchtet werden, was bei Ressourcenknappheit weiteren apparativen Aufwand für eine Atemluftkonditionierung eliminieren würde.

Herkömmliche Beatmungsgeräte, insbesondere für intensivmedizinische Zwecke, sind somit typischerweise kostenintensive, komplexe Geräte, die jeweils speziell für die Anforderungen einer konkreten Anwendung, wie z. B. in der Klinik oder in einem Rettungsfahrzeug, ausgelegt sind. Der Betrieb herkömmlicher Beatmungsgeräte erfordert in der Regel die Einbindung in vorhandene intensivmedizinische Technik und die Bedienung und Überwachung durch spezialisiertes Personal.

Durch pandemisch auftretende Lungenkrankheiten, wie den Covid-19-Ausbruch im Jahr 2020, entsteht ein Bedarf an Beatmungsgeräten mit verminderten Kosten, minimalem technischen Aufwand, minimaler Bindung klinischer Ressourcen und einem möglichst breiten Anwendungsbereich.

Die Aufgabe der Erfindung ist es, eine verbesserte Beatmungsvorrichtung mit einem Radialgebläse bereitzustellen, mit der Nachteile herkömmlicher Techniken vermieden werden. Die Beatmungsvorrichtung soll insbesondere ohne Funktionseinschränkung bei ihrer Anwendung einen vereinfachten Aufbau haben, mit verminderten Kosten herstellbar sein, eine verringerte Leistungsaufnahme haben, möglichst unabhängig von weiteren Geräten betreibbar sein, automatisiert betreibbar sein und/oder einfach bedienbar sein.

Diese Aufgabe wird durch eine Beatmungsvorrichtung gelöst, welche die Merkmale des unabhängigen Anspruchs aufweist. Bevorzugte Ausführungsformen und Anwendungen der Erfindung ergeben sich aus den abhängigen Ansprüchen.

Gemäß einem ersten allgemeinen Gesichtspunkt der Erfindung wird die genannte Aufgabe durch eine Beatmungsvorrichtung gelöst, die zur nicht-invasiven Beatmung einer Person eingerichtet ist. Die Beatmungsvorrichtung umfasst ein Radialgebläse mit einer Einlassöffnung und einer Auslassöffnung. Das Radialgebläse ist zur Aufnahme von Beatmungsluft an der Einlassöffnung und zur Bereitstellung der Beatmungsluft mit einem einstellbaren Beatmungsdruck an der Auslassöffnung eingerichtet. Der Beatmungsdruck ist vorzugsweise durch Einstellung der Leistung des Radialgebläses, insbesondere der Drehzahl des Radialgebläses, einstellbar. In verschiedenen Atmungsphasen sind verschiedene Beatmungsdrucke einstellbar.

Die Beatmungsvorrichtung umfasst des Weiteren eine erste Atemleitung (oder: peripherieseitige Atemleitung) mit einem ersten Ende, das mit der Einlassöffnung des Radialgebläses verbunden ist, und mit einem zweiten Ende, das zur Aufnahme von Einatemluft angeordnet ist. Weiterhin umfasst die Beatmungsvorrichtung eine zweite Atemleitung (oder: patientenseitige Atemleitung) mit einem ersten Ende, das mit der Auslassöffnung des Radialgebläses verbunden ist, und mit einem zweiten Ende, das für eine Ankopplung einer Atemmaske eingerichtet ist. Vorzugsweise ist das zweite Ende der zweiten Atemleitung mit einer Kopplungseinrichtung ausgestattet, die für eine Verbindung mit der Atemmaske eingerichtet ist. Alternativ ist das zweite Ende der zweiten Atemleitung mit der Atemmaske fest verbunden. Die Gesichtsmaske kann ein Teil der Beatmungsvorrichtung sein. Die Atemmaske ist eine Gesichtsmaske, die auf dem Gesicht einer beatmeten Person fixierbar ist. Vorzugsweise ist eine nicht zusätzlich belüftete Atemmaske vorgesehen, die eine Gesichtsmaske umfasst, die luftdicht auf dem Gesicht der beatmeten Person dicht sitzt, so dass bei ungestörtem Betrieb und korrektem Sitz die Person ausschließlich über die patientenseitige Atemleitung atmet und keine oder vernachlässigbar wenig Luft seitlich ein- oder austritt.

Die zweite Atemleitung ist mit einer Sensoreinrichtung ausgestattet, mit der mindestens ein Strömungsparameter in der zweiten Atemleitung messbar ist. Eine Steuereinrichtung der Beatmungsvorrichtung, vorzugsweise mindestens eine Mikroprozessor-Schaltung, ist zur Steuerung des Radialgebläses in Abhängigkeit von mindestens einem Ausgangssignal der Sensoreinrichtung vorgesehen.

Gemäß der Erfindung bilden das Radialgebläse und die zweite Atemleitung einen durchgehenden bidirektionalen Strömungsweg, d. h. ein Strömungsweg mit zwei abwechselnden Strömungsrichtungen. Das Radialgebläse und die zweite Atemleitung sind jeweils in den verschiedenen Atmungsphasen, insbesondere der Einatmungsphase und der Ausatmungsphase, für entgegengesetzte Strömungsrichtungen ausgelegt. Optional ist auch die erste Atemleitung oder zumindest ein Teil von dieser bis zu einer optional vorgesehenen Ventileinrichtung Teil des durchgehenden bidirektionalen Strömungswegs. Die Strömungsrichtungen umfassen eine erste Strömungsrichtung von dem zweiten Ende der ersten Atemleitung über das Radialgebläse zu dem zweiten Ende der zweiten Atemleitung (Einatmungsphase) oder eine zweite Strömungsrichtung von dem zweiten Ende der zweiten Atemleitung über das Radialgebläse zu dem zweiten Ende der ersten Atemleitung (Ausatmungsphase) oder optional zu einem Exspirationsventil zwischen dem Radialgebläse und dem ersten Ende der ersten Atemleitung. Der einstellbare Beatmungsdruck an der Auslassöffnung des Radialgebläses ist ein Einatmungsdruck oder ein Ausatmungsdruck, der gleich dem oder geringer als der Einatmungsdruck ist.

Der Erfinder hat festgestellt, dass die Ausatmung durch das Radialgebläse und gegen den Arbeitsdruck des Radialgebläses erfolgen kann, wodurch die Bereitstellung des bidirektionalen Strömungswegs ermöglicht wird. Der bidirektionale Strömungsweg bietet den Vorteil, dass der Aufbau der Beatmungsvorrichtung im Vergleich zu herkömmlichen Techniken erheblich vereinfacht wird. Die patientenseitige Atemleitung kann eine einzige Atemleitung umfassen, über welche die Beatmungsvorrichtung mit der Atemmaske verbunden ist. Die Bereitstellung des bidirektionalen Strömungswegs ermöglicht des Weiteren eine vereinfachte Sensorik. Mit der lokal auf die zweite Atemleitung konzentrierten Sensoreinrichtung können alle zur Steuerung der Beatmungsvorrichtung erforderlichen Strömungsparameter erfasst werden, wodurch die Steuerung der Beatmungsvorrichtung vereinfacht wird.

Des Weiteren ist gemäß der Erfindung die zweite Atemleitung mit einer Filtereinrichtung ausgestattet, die zur Luftströmungsfilterung angeordnet ist. Die Filtereinrichtung umfasst einen Filter, bevorzugt einen HEPA-Filter, durch den die Strömung in der zweiten Atemleitung durchtritt. Vorteilhafterweise erfüllt die Filtereinrichtung durch die Bereitstellung des bidirektionalen Strömungswegs eine Doppelfunktion. Erstens wird die Umgebung vor Keimen geschützt, die ggf. aus dem Patienten austreten könnten, und zweitens wird der Patient vor Keimen geschützt, die ggf. aus der Umgebung einwandern könnten. Durch die Bereitstellung einer einzigen Filtereinrichtung, vorzugsweise mit einem einzigen Filter, werden der Aufbau und der Betrieb der Beatmungsvorrichtung weiter vereinfacht.

Vorteilhafterweise ist das Radialgebläse miniaturisierbar, so dass mit der erfindungsgemäßen Beatmungsvorrichtung ein System geschaffen wird, das einfach herstellbar und in der Lage ist, einen automatisierbaren Beatmungsprozess mit minimalem technischen Aufwand (vorzugsweise Atemmaske, HEPA-Filter und mikroprozessor-gesteuertes Radialgebläse) mit einem Durchmesser gleich oder geringer 10 cm, vorzugsweise gleich oder geringer 7 cm, und einer Masse gleich oder geringer 100 g, vorzugsweise gleich oder geringer 60 g, zu realisieren. Des Weiteren wird eine äußerst geringe Leistungsaufnahme von beispielsweise ca. 12 W ermöglicht, was einen längeren Betrieb an einer Batterie, wie z. B. einer Fahrzeugbatterie, ermöglicht, um auch bei instabiler elektrischer Versorgung, z.B. unter Katastrophenbedingungen, die Funktionsfähigkeit aufrechtzuerhalten.

Offenbart ist auch ein Steuerverfahren zur Steuerung eines Radialgebläses zur Beatmung einer Person.

Das Steuerverfahren umfasst die Schritte Steuerung des Radialgebläses mit einem ersten Regelkreis, mit dem ein Beatmungsdruck an einer Auslassöffnung des Radialgebläses auf einen Einatmungsdruck oder auf einen Ausatmungsdruck einstellbar ist, der gleich dem oder geringer als der Einatmungsdruck ist, und Steuerung des Radialgebläses mit einem zweiten Regelkreis, mit dem mindestens eines einstellbar ist von einer Frequenz von Einatmungsphasen, in denen der Einatmungsdruck an der Auslassöffnung des Radialgebläses gebildet ist, und einem Tastverhältnis der Dauer der Einatmungsphasen relativ zu der Dauer von Ausatmungsphasen, in denen der Ausatmungsdruck an der Auslassöffnung des Radialgebläses gebildet ist.

Die Steuerung des Radialgebläses umfasst die Einstellung einer Zeitfunktion der Gebläsedrehzahl des Radialgebläses. Das Steuerverfahren umfasst mit der Implementierung der zwei Regelkreise ein Regelverfahren, das basierend auf Ausgangswerten der Sensoreinrichtung (Istwerte) vorgegebene Strömungsparameter (Sollwerte) einstellt, um die genannten Größen Einatmungsdruck, Ausatmungsdruck und Frequenz von Einatmungsphasen und/oder Tastverhältnis der Dauer der Einatmungsphasen relativ zu der Dauer von Ausatmungsphasen zu erhalten. Vorzugsweise wird mit dem Steuerverfahren oder einem seiner Ausführungsformen das Radialgebläse der Beatmungsvorrichtung gemäß dem ersten allgemeinen Gesichtspunkt der Erfindung oder einer ihrer Ausführungsformen gesteuert.

Offenbart ist auch ein Verfahren zum Betrieb einer Beatmungsvorrichtung gemäß dem ersten allgemeinen Gesichtspunkt der Erfindung oder einer ihrer Ausführungsformen, wobei das Verfahren die Schritte Betrieb des Radialgebläses, Messung mindestens eines Strömungsparameters mit der Sensoreinrichtung, und Steuerung des Radialgebläses mit einem Steuerverfahren gemäß dem zweiten allgemeinen Gesichtspunkt der Erfindung oder einer seiner Ausführungsformen umfasst. Mit dem Verfahren zum Betrieb der Beatmungsvorrichtung wird vorzugsweise ein Beatmungsverfahren zur Beatmung einer Person, die eine mit der Beatmungsvorrichtung gekoppelte Gesichtsmaske trägt, unter Schutz gestellt.

Der Erfinder hat festgestellt, dass die Regelung des Radialgebläses ermöglicht, alle gewünschten Funktionen einer Beatmungsvorrichtung zu erfüllen und mit dem Steuerverfahren und dem Beatmungsverfahren kontinuierlich das gesamte Spektrum zwischen reiner Spontanatmung über Spontanatmungsunterstützung bis in den Bereich rein automatischer Beatmung bei vollständiger respiratorischer Insuffizienz abzudecken. Der durch die Beatmungsvorrichtung generierte Beatmungsdruck hängt vorteilhafterweise nicht oder vernachlässigbar wenig vom Fluss (Amplitude und/oder Richtung) ab, so dass die bidirektionale Spontanatmung des Patienten einerseits nicht behindert und andererseits durch Flussänderung einfach detektiert werden kann. Unter dieser Voraussetzung ergeben sich auch einfache Indizien, um die Beatmungsvorrichtung auf die Spontanatmung des Patienten synchronisieren zu können (so genanntes offenes System).

Vorteilhafterweise kann das Tidalvolumen bzw. Minutenvolumen der Atmung durch eine Einstellung des Beatmungsdrucks (Inspirationsdrucks) (IPAP) und der Beatmungsfrequenz (f) bzw. des Inspirations-Exspirations-Verhältnis (I:E-Verhältnis) angepasst werden. Typische obere Grenzwerte sind z. B. IPAP < 30mbar, f < 25/min und I:E < 1.

Vorteilhafterweise erlaubt die Sensoreinrichtung in der zweiten Atemleitung, insbesondere den Druck als innerste Führungsgröße der genannten Regelkreise mit hoher Genauigkeit (besser 0.5 mbar) und des Weiteren die Strömungsgeschwindigkeit mit hoher Genauigkeit (besser +/- 0.2 l/min) zu messen.

Gemäß einer bevorzugten Ausführungsform der Erfindung ist der Strömungsweg zwischen dem zweiten Ende der zweiten Atemleitung und der Einlassöffnung des Radialgebläses frei von Ventilen. Der Strömungsweg entlang der zweiten Atemleitung ist insbesondere verzweigungsfrei. Vorteilhafterweise wird damit ermöglicht, dass die Beatmungsvorrichtung ohne Doppelschlauch und ohne Exspirationsventil zwischen Gesichtsmaske und Radialgebläse aufgebaut ist, wodurch das Schlauchvolumen der zweiten Atemleitung insbesondere zwischen der Filtereinrichtung und dem Radialgebläse und entsprechend die Rückatmung von $CO_2$ minimiert werden können.

Gemäß einer weiteren bevorzugten Ausführungsform der Erfindung weist die erste Atemleitung zwischen der Einlassöffnung des Radialgebläses und dem zweiten Ende der ersten Atemleitung eine Ventileinrichtung, insbesondere ein passives differenzdruckgesteuertes Auslassventil oder ein aktiv betätigbares Dreiwegeventil, auf. Die Ventileinrichtung ist als ein Exspirationsausgang zum Auslass von Ausatemluft angeordnet. Vorteilhafterweise kann ein einfaches, passives Exspirationsventil in der ersten Atemleitung, besonders bevorzugt unmittelbar vor dem ersten Ende der ersten Atemleitung (Saugeingang des Radialgebläses), angeordnet sein. Ein passives Exspirationsventil ist ein Ausatemventil, das bei innerem Ausatemdruck in der ersten Atemleitung über dem Atmosphärendruck und einer Flussrichtung hin Auslass in die Umgebung öffnet und im Übrigen geschlossen ist, wie z. B. ein differenzdruckgesteuertes Folienventil. Der Saugeingang des Radialgebläses wird somit vorteilhafterweise automatisch je nach Richtung des durch das Radialgebläse fließenden Luftstromes zwischen Eingang zur Zufuhr von Sauerstoff (ggf. mit Befeuchtung) und Exspirationsausgang umgeschaltet. Damit wird eine besonders einfache Trennung von Inspirations- und Exspirationsluft bereitgestellt. Das passive automatische Umschalten wird insbesondere durch den Strömungswiderstand der ersten Atemwegsleitung und einer optional vorgesehenen Atemluftkonditionierungseinrichtung bestimmt.

Vorteilhafterweise bestehen verschiedene Möglichkeiten, über die erste Atemleitung Atemluft für den Patienten aufzunehmen. Gemäß einer ersten Variante kann sich das zweite Ende der ersten Atemleitung in eine Umgebung der Beatmungsvorrichtung öffnen. Damit wird der Aufbau der Beatmungsvorrichtung vorteilhafterweise besonders kompakt und leicht. Alternativ oder zusätzlich kann gemäß einer zweiten Variante das zweite Ende der ersten Atemleitung für eine Ankopplung an ein Atemluftreservoir, wie z. B. eine Druckluftflasche mit einem Druckreduzierventil, eingerichtet sein. In diesem Fall ergeben sich Vorteile aus der definierten Zufuhr von Atemluft mit einem vorbestimmten Sauerstoffanteil bis hin zu reinem Sauerstoff.

Vorteilhafterweise ist gemäß einer weiteren Ausführungsform der Erfindung eine Atemluftkonditionierungseinrichtung mit dem zweiten Ende der ersten Atemleitung gekoppelt. Die Atemluftkonditionierungseinrichtung ist eine an das Innere der ersten Atemleitung angrenzende Komponente, die durch physikalische und/oder chemische Wechselwirkung die Zusammensetzung und/oder physikalische Eigenschaften der in der ersten Atemleitung strömenden Atemluft einstellt. Bevorzugt ist die Atemluftkonditionierungseinrichtung zur Einstellung von mindestens einem der Parameter Feuchte, Temperatur und Sauerstoffgehalt der Einatemluft eingerichtet. Hierzu umfasst die Atemluftkonditionierungseinrichtung vorzugsweise eine steuerbare Zufuhreinheit für die Einleitung von Wasserdampf oder wässrigem Aerosol, ggf. versetzt mit pharmazeutischen Wirkstoffen, eine Temperierungseinrichtung (Heiz- und/oder Kühleinrichtung), z. B. basierend auf einem Widerstandsheizelement und/oder einem Peltier-Element, bzw. eine steuerbare Zufuhreinheit für die Einleitung von Sauerstoff. Die Atemluftkonditionierungseinrichtung kann z. B. ein Verdunstungssystem mit steriler Wasserzuführung aus Einwegbeuteln, wie es im klinischen Bereich durch die Infusionstechnik bekannt ist, oder eine kommerziell verfügbare Befeuchtereinheit umfassen.

Die Atemluftkonditionierungseinrichtung bietet den Vorteil, dass die Akzeptanz einer Beatmung und das Wohlbefinden des Patienten erhöht werden können, wenn das Atemgas befeuchtet und/oder temperiert wird, insbesondere wenn der Patient schon bei verspürter Atemnot reflexartig im Wesentlichen durch den Mund atmet. Die bei einer herkömmlichen Beatmungsvorrichtung unzulängliche Funktion eines HME-Filters kann von der Atemluftkonditionierungseinrichtung erfüllt werden. Die Temperierung kann eine Erwärmung der Atemleitungen und des Radialgebläses mittels der temperierten Atemluft auf mindestens 30 °C, vorzugsweise mindestens 35° C, bis zu einer physiologisch akzeptablen Temperatur umfassen. Vorteilhafterweise wird damit nach der Befeuchtung Kondensat und Keimbildung im System verhindert.

Die Temperierungseinrichtung kann zur unmittelbaren Temperierung der Atemluft und/oder zur unmittelbaren Temperierung der Beatmungsvorrichtung, insbesondere der zweiten Atemleitung, eingerichtet sein. Durch die Temperierung der Beatmungsvorrichtung werden die Bildung von Kondensat und Keimen besonders wirksam verhindert.

Wenn gemäß einer weiteren Variante der Erfindung die Filtereinrichtung eine Steckverbindung mit dem Radialgebläse und/oder der zweiten Atemleitung aufweist, ergeben sich Vorteile für die Anwendung der Beatmungsvorrichtung, insbesondere für einen einfachen Filterwechsel. Die Beatmungsvorrichtung kann nach Gebrauch bei einem Patienten leicht durch einen Filterwechsel und eine Desinfektion der übrigen Teile für einen neuen Patienten vorbereitet werden. Die Steckverbindung kann verriegelbar sein, z. B. einen Bajonettverschluss aufweisen, um eine unbeabsichtigte Trennung der Teile der Beatmungsvorrichtung zu vermeiden.

Gemäß einer vorteilhaften Ausführungsform der Erfindung ist die Steuereinrichtung mit einer Schnittstelle zur Aufnahme eines Ausgangssignals eines Sauerstoffsättigungssensors ausgestattet. Vorzugsweise ist ein Sauerstoffsättigungssensor mit der Beatmungsvorrichtung fest verbunden. Alternativ kann ein separater Sauerstoffsättigungssensor eines Peripheriegerätes verwendet werden. Der Sauerstoffsättigungssensor ist ein an sich bekannter chemischer und/oder optischer Sensor, mit dem als zentrale Führungsgröße die Sauerstoffsättigung (SpO2) im Blut der beatmeten Person messbar ist.

Vorzugsweise ist die Steuereinrichtung unmittelbar an das Radialgebläse angrenzend angeordnet. Wenn die Steuereinrichtung insbesondere direkt auf dem Radialgebläse sitzt, werden vorteilhafterweise die Schnittstellen zu der Sensoreinrichtung und zu Aktuatoren der Beatmungsvorrichtung minimiert.

Neben der Steuerfunktion kann die Steuereinrichtung vorteilhafterweise eine Alarmfunktion erfüllen und insbesondere zur Ausgabe eines Alarmsignals bei Feststellung einer Fehlfunktion der

Beatmungsvorrichtung und/oder eines bedenklichen Zustands der beatmeten Person eingerichtet sein. Das Alarmsignal kann z. B. ein Lichtsignal und/oder ein Schallsignal und/oder einen digitalen Alarm (Alarmsignal, das über eine Netzwerkverbindung zu einem zentralen Überwachungscomputer übermittelt wird) umfassen. Mit dem Alarmsignal kann insbesondere dem Klinikpersonal signalisiert werden, wenn eine Destabilisierung der Beatmung droht.

Die Steuereinrichtung enthält insbesondere eine Motorsteuerung, wie z. B. einen BLDC-Controller, die vorzugsweise unmittelbar am Motor angeordnet ist, um elektromagnetische Störungen in der Umgebung zu minimieren.

Die Steuereinrichtung der Beatmungsvorrichtung ist zur Steuerung des Radialgebläses mit dem ersten Regelkreis (innerster Regelkreis) eingerichtet, mit dem der Beatmungsdruck an der Auslassöffnung des Radialgebläses einstellbar ist. Der erste Regelkreis bildet eine Druckregelung. Wenn das Radialgebläse gemäß einer bevorzugten Variante eine flache Kennlinie besitzt, ist der Beatmungsdruck proportional zum Quadrat der Drehzahl des Radialgebläses. Der Beatmungsdruck besitzt eine verschwindende oder vernachlässigbare Abhängigkeit von der Strömungsgeschwindigkeit, so dass der erste Regelkreis vorzugsweise einen PI-Regler mit zwei angelernten I-niveaus für PIP und PEEP als Sollwerte umfasst.

Des Weiteren ist die Steuereinrichtung zur Steuerung des Radialgebläses mit dem zweiten Regelkreis eingerichtet, mit dem mindestens eines von einer Frequenz der Einatmungsphasen, in denen der Einatmungsdruck an der Auslassöffnung des Radialgebläses gebildet ist, und einem Tastverhältnis der Dauer der Einatmungsphasen relativ zu der Dauer von Ausatmungsphasen, in denen der Ausatmungsdruck an der Auslassöffnung des Radialgebläses gebildet ist, einstellbar ist. Die nächste höhere Regelstufe über dem ersten Regelkreis wird somit durch eine Zeitsteuerung gebildet, deren Sollwerte für Frequenz (f) und Tastverhältnis (I:E) innerhalb der physiologisch sinnvollen Grenzen eingestellt und parametrisierbar sind. Mit dem zweiten Regelkreis werden der Druckregelung im ersten Regelkreis die Umschaltzeitpunkte vorgegeben. Vorzugsweise enthält der zweite Regelkreis auch eine Anpassungsfunktion, um den Betrieb des Radialgebläses auf die Spontanatmungsbemühungen des Patienten zu synchronisieren.

Gemäß einer besonders bevorzugten Ausführungsform der Erfindung ist die Steuereinrichtung zur Steuerung des Radialgebläses zusätzlich mit einem dritten Regelkreis eingerichtet, mit dem Sollgrößen des ersten und/oder des zweiten Regelkreises derart einstellbar sind, dass das Tidalvolumen und/oder das Minutenvolumen der Beatmung an vorbestimmte Referenzgrößen des Tidalvolumens bzw. des Minutenvolumen angepasst werden. Die physiologisch vorgegebenen Referenzgrößen werden durch die Steuerung mit dem dritten Regelkreis angestrebt, d. h. das Tidalvolumen bzw. das Minutenvolumen werden auf die Referenzgrößen des Tidalvolumens bzw. des Minutenvolumen eingestellt oder wenigstens an diese angenähert. Der dritte Regelkreis bildet ein weiteres Regelungslevel, mit dem vorteilhafterweise auf Änderungen der Steifigkeit der Lunge (Compliance) und den Atemwegswiderstand (Resistance) innerhalb vorgegebener, parametrisierbarer Grenzen reagieren werden kann. Führungsgröße des dritten Regelkreises ist das durch zeitliche Integration des Flusses während Inspirations- und Exspirationsphasen errechnete Tidalvolumen. Der Sollwert für diesen Regler wird durch den Parameter Minutenvolumen vorgegeben, der mit der Sensoreinrichtung erfassbar ist. Der Ausgang des dritten Regelkreises modifiziert die Sollwertvorgaben der Druckregelung und der Zeitsteuerung. Somit ergibt sich eine indirekt volumenkontrollierte Beatmung, wie sie bei den Beatmungsverfahren MMV (Mandatory Minute Volume) und Dräger AutoFlow vorgesehen ist, ohne die potentiell schädlichen Nebenwirkungen von herkömmlichen direkten Volumenkontrollverfahren.

Besonders bevorzugt ist die Steuereinrichtung des Weiteren zur Steuerung des Radialgebläses in Abhängigkeit von einem Ausgangssignal eines Sauerstoffsättigungssensors mit einem vierten Regelkreis eingerichtet, mit dem eine Sollgröße des dritten Regelkreises derart einstellbar ist, dass die Sauerstoffsättigung im Blut der beatmeten Person an eine vorbestimmte Sauerstoffsättigungs-Referenzgröße angepasst wird. Die Sauerstoffsättigungs-Referenzgröße ist physiologisch vorgegeben und wird durch die Steuerung mit dem vierten Regelkreis angestrebt, d. h. die Sauerstoffsättigung wird auf die Sauerstoffsättigungs-Referenzgröße eingestellt oder wenigstens an diese angenähert. Auf diesem höchsten Regelungslevel wird der Sollwert des Minutenvolumens durch einen i-Regler vorgegeben, der die Sauerstoffsättigung als Führungsgröße hat, und mit seinem lokalen Sollwert vergleicht. Hiermit wird die Gesamtregelung vorteilhafterweise geschlossen.

Weitere Einzelheiten und Vorteile der Erfindung werden im Folgenden unter Bezug auf die beigefügten Zeichnungen beschrieben. Die Zeichnungen zeigen schematisch in

Figur 1: eine Perspektivansicht einer bevorzugten Ausführungsform der erfindungsgemäßen Beatmungsvorrichtung;

Figur 2: Seitenansichten der Beatmungsvorrichtung gemäß Figur 1;

Figur 3: Schnittansichten der Beatmungsvorrichtung entlang den Linien 3A und 3B in Figur 2;

Figur 4: ein Strömungsdiagramm der Beatmungsvorrichtung gemäß bevorzugten Ausführungsformen der Erfindung; und

Figur 5: ein Flussdiagramm einer Steuerung eines Radialgebläses einer Beatmungsvorrichtung.

Merkmale bevorzugter Ausführungsformen der Erfindung werden im Folgenden beispielhaft unter Bezug auf eine Konfiguration der Beatmungsvorrichtung in einem kugelförmigen Gehäuse, ein Strömungsdiagramm der Beatmungsvorrichtung und ein Flussdiagramm der bevorzugt vorgesehenen Regekreise beschrieben. Es wird betont, dass die Umsetzung der Erfindung in der Praxis nicht auf die gezeigten Beispiele beschränkt, sondern insbesondere in Bezug auf die Dimensionen, Formen, Materialien und Gestaltung der Regelkreise abgewandelt werden kann.

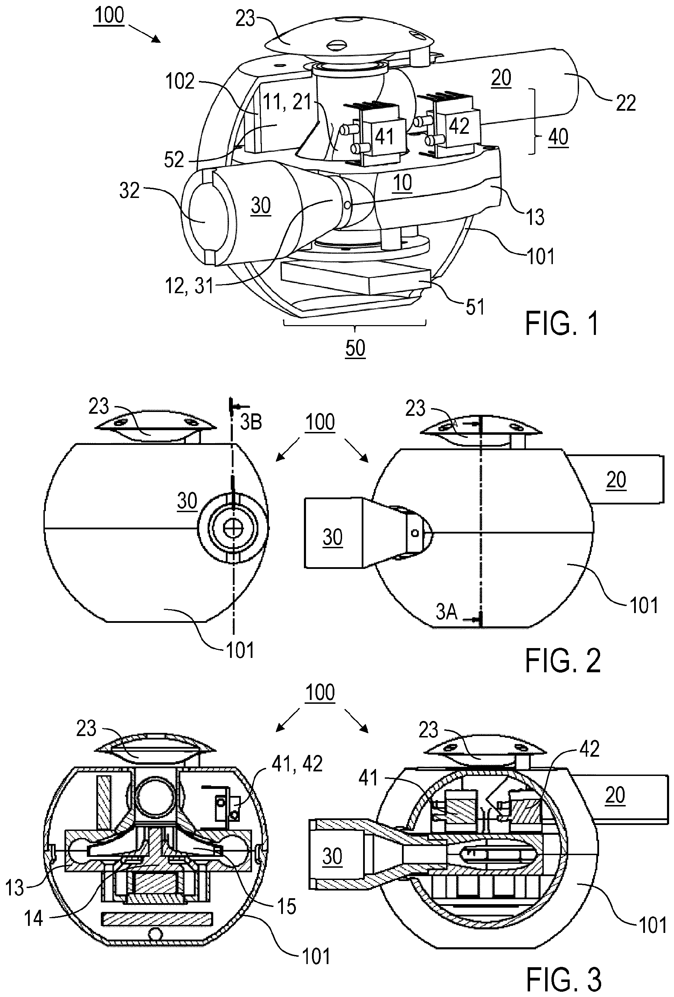

Die Beatmungsvorrichtung 100 ist in Figur 1 in Perspektivansicht und in den Figuren 2 und 3 in Seiten- und Schnittansichten mit dem Radialgebläse 10, der ersten Atemleitung 20 (teilweise dargestellt), der zweiten Atemleitung 30 (teilweise dargestellt), der Sensoreinrichtung 40 und der Steuereinrichtung 50 in einem im Wesentlichen kugelförmigen Gehäuse 101 schematisch dargestellt. Das Radialgebläse 10 enthält in an sich bekannter Weise in einem Gebläsegehäuse 13 einen Elektromotor 14 und einen Impeller mit Gebläseschaufeln 15 (siehe Figur 3A). Der Elektromotor 14 ist z. B. ein bürstenloser Gleichstrommotor (BLDC-Motor). Die Drehachse des Elektromotors 14 definiert eine Hauptachse (z-Richtung) der Beatmungsvorrichtung 100. Das Gebläsegehäuse 13 erstreckt sich senkrecht zur Hauptachse, und es weist eine mittige, sich entlang der Hauptachse öffnende Einlassöffnung 11 und eine tangentiale, sich senkrecht zur Hauptachse öffnende Auslassöffnung 12 auf. Mit der Einlassöffnung 11 ist das erste Ende 21 der ersten Atemleitung 20 verbunden und mit der Auslassöffnung 12 ist das erste Ende 31 der zweiten Atemleitung 30 verbunden.

Bei Betrieb des Radialgebläses 10 wird über die Einlassöffnung 11 Beatmungsluft angesaugt und an der Auslassöffnung 12 mit einem von der Atmungsphase abhängigen Beatmungsdruck bereitgestellt. Vorzugsweise ist das Radialgebläse 10 mit geraden, radial verlaufenden Gebläseschaufeln 15 ausgestattet, wobei zum Druckaufbau die Zentripetalwirkung der Gebläseschaufeln 15 und keine aerodynamischen Effekte benutzt werden. Besonders bevorzugt sind die Gebläseschaufeln 15 in Bezug auf die Umfangsrichtung des Radialgebläses schräg gestellt. Damit wird zwar die Effektivität, jedoch auch das Betriebsgeräusch des Gebläses verringert. Bei einer durch die Betriebsspannung gegebenen Maximaldrehzahl von 40.000 Umdrehungen/min wird z. B. ein maximaler Beatmungsdruck von etwa 35 mbar bei einem Fluss von maximal etwa 100 l/min erzeugt.

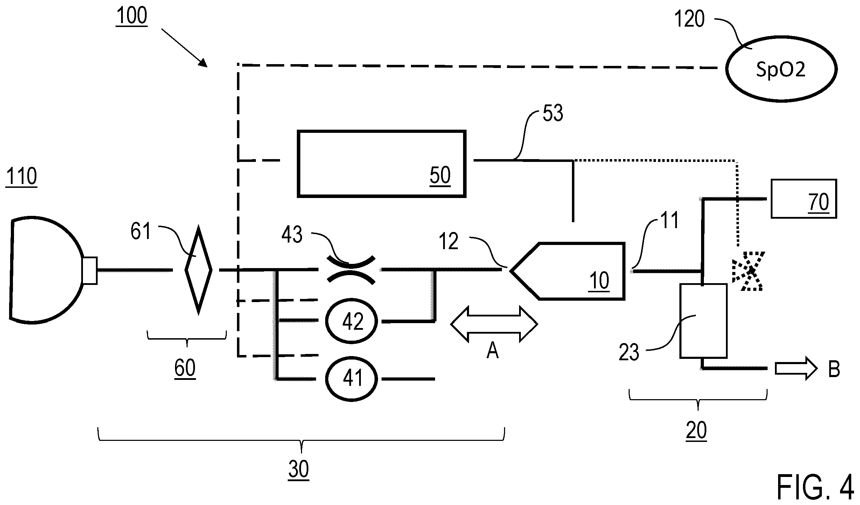

Die erste Atemleitung 20 und die zweite Atemleitung 30 sind jeweils aus einem starren oder flexiblen Leitungsmaterial gebildet und umfassen vorzugsweise jeweils ein Kunststoffrohr. Das zweite Ende 22 der ersten Atemleitung 20 ist mit der Atemluftkonditionierungseinrichtung 70 verbunden und zur Aufnahme von Einatmungsluft angeordnet (siehe Figur 4). Das zweite Ende 32 der zweiten Atemleitung 30 ist über eine Filtereinrichtung 60 mit der Gesichtsmaske 110 verbunden (siehe Figur 4).

Direkt an der Einlassöffnung 11 des Radialgebläses 10 ist an dem zweiten Ende 22 der ersten Atemleitung 20 die Ventileinrichtung 23, umfassend ein passives Auslassventil, vorgesehen. Die Ventileinrichtung 23 schließt in den Einatmungsphasen, so dass Einatmungsluft zum Radialgebläse 10 zugeführt wird, und sie öffnet bei einem erhöhten Druck in Ausatmungsphasen, so dass Ausatemluft an der der ersten Atemleitung 20 vorbei in die Umgebung abgeleitet wird. Das Auslassventil ist vorzugsweise ein einfaches Exspirationsventil, das direkt vor der Einlassöffnung des Gebläses 11 sitzt. Die Einlassöffnung des Gebläses 11 wird damit je nach Vorzeichen des durch das Radialgebläse 10 fließenden Luftstromes zwischen Sauerstoff / Befeuchtung und Exspirationsausgang umgeschaltet.

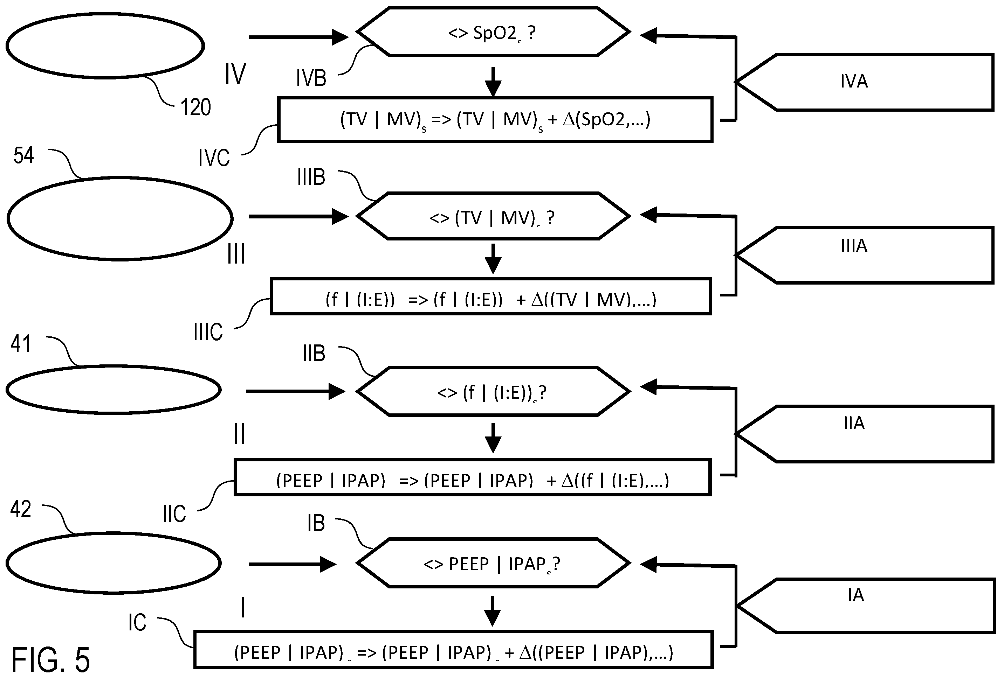

Die Sensoreinrichtung 40 umfasst einen Drucksensor 41 und einen Flusssensor 42, mit denen der Druck bzw. die Strömungsgeschwindigkeit in der zweiten Atemleitung 30 erfassbar sind. Die Messung erfolgt an einer Verengung 43 in der zweiten Atemleitung 30 (siehe Figur 4). Die Verengung 43 umfasst z. B. eine Düse. Der Drucksensor 41 und der Flusssensor 42 sind mit der Steuereinrichtung 50 verbunden, die das Radialgebläse 10 in Abhängigkeit von Ausgangssignalen der Sensoren regelt, wie unten unter Bezug auf Figur 5 beschrieben ist.

Die Steuereinrichtung 50 umfasst, wie schematisch in Figur 1 gezeigt ist, eine Motorsteuerung 51 für die Bereitstellung eines Steuersignals für den Betrieb des Elektromotors 14 und eine Steuerschaltung 52 zur Realisierung der Regelkreise für die Regelung des Radialgebläses 10. Die Motorsteuerung 51, wie z. B. ein BLDC-Controller, ist vorzugsweise direkt am Elektromotor 14 angeordnet, um elektromagnetische Störungen (EMI) zu minimieren. Die Steuerschaltung 52, wie z. B. ein Arduino Microcontroller, sitzt zur Steuerung der Beatmungsvorrichtung 100 vorzugsweise direkt auf dem Radialgebläse 10, um die Schnittstellen zu den Sensoren und Aktuatoren zu minimieren.

Die Beatmungsvorrichtung 100 ist des Weiteren mit einer elektrischen Schnittstelle 102 ausgestattet (siehe Figur 1), die für den Anschluss von mindestens einer elektrischen Verbindung zur Energieversorgung und zum Datenaustausch und/oder für eine drahtlose Verbindung der Beatmungsvorrichtung 100 mit einem externen Netzwerk oder Steuergerät konfiguriert ist. Die Schnittstelle 102 ist z. B. für ein 4-poliges Kabel (2 Pole für Versorgungsspannung, z. B. 12V, und 2 Pole für einen USB-Port) ausgelegt und mit einer Schnittstelle für den $SpO_2$-Sensor, einer Schnittstelle für einen optischen und/oder akustischen Alarm und einer Bluetooth-Schnittstelle für eine Fernbedienung und/oder einen Datenlogger, z. B. mit einem Computer oder Smartphone ausgelegt.

Im Folgenden wird die pneumatische Konfiguration des Radialgebläses 10 beschrieben, das als Druckerzeuger zur Erzeugung eines flussunabhängigen Beatmungsdrucks von z. B. 5 bis 30 mbar arbeitet. Mit dem Radialgebläse 10 mit geraden, radial verlaufenden Gebläseschaufeln 15 ergibt sich der Beatmungsdruck an der Auslassöffnung des Radialgebläses 10 allgemein gemäß

$$p = 1/2 * \rho * (2*pi*f)^2 * (r_a^2 - r_i^2)$$

mit: $\rho$ = Dichte der Luft, f = Drehzahl, $r_a$ = Außendurchmesser des Impellers, $r_i$ = Innendurchmesser des Impellers. Mit einer mit einem BLDC-Motor realisierten Maximaldrehzahl von 40.000 U/min (670 Hz), ergibt sich ein minimaler Außendurchmesser von etwa 40 mm. Um einen Fluss von z. B. 100 l/min erreichen zu können, weisen die Gebläseschaufeln 15 auf dem Umfang vorzugsweise eine Höhe von etwa 4 mm auf. Mit dem Gebläsegehäuse 13 ergibt sich eine Größe des Radialgebläses 10 von etwa 60 mm Durchmesser und etwa 15 mm Höhe bei einer Masse von etwa 20 g. Das Totvolumen des Radialgebläses 10 beträgt nur etwa 10 ml.

Mit diesen beispielhaften Parametern ergibt sich eine mittlere Leistung P gemäß P = 0.001 $m^3$/s * 2.500 Pa = 2.5 W. Bei einem angenommenen Wirkungsgrad der Beatmungsvorrichtung 100 von 25% ergibt sich die mittlere, vom Elektromotor 14 aufgenommene Leistung von ca. 10 W bis 12 W. Ein Motor dieser Leistungsklasse wiegt etwa 10 g und hat einen Durchmesser von etwa 18 mm und eine Länge von etwa 15 mm. Der Formfaktor des Gebläses (Durchmesser >> Länge) ermöglicht die Integration des Gebläses, der Messstrecke für die Flussmessung und die Druckmessung, die Motorsteuerung und das Exspirationsventil in ein kugelförmiges Gehäuse 101 mit der Größe eines Tennisballs und einer Masse von etwa 50 g. Vorteilhafterweise werden damit vergleichbare Größen wie mit einem Atemgasfilter vom Typ Pall Ultipor 50 erreicht, der eine Masse von 26 g (trocken) und 35 ml Totvolumen aufweist.

Das Strömungsdiagramm gemäß Figur 4 illustriert schematisch die pneumatische und elektrische Konfiguration der Beatmungsvorrichtung 100 mit dem Radialgebläse 10, den ersten und zweiten Atemleitungen 20, 30, der Sensoreinrichtung 40, der Steuereinrichtung 50, der Filtereinrichtung 60 und der Atemluftkonditionierungseinrichtung 70. Die pneumatischen Schnittstellen des Radialgebläses 10 umfassen die Einlassöffnung 11 mit einem Schlauchanschluss für die Luftzufuhr und mit dem Exspirationsventil der Ventileinrichtung 23, und die Auslassöffnung 12, die sich zur Sensoreinrichtung 40 und der Filtereinrichtung 60 öffnet. Es wird der durchgehende bidirektionale Strömungsweg (siehe Doppelpfeil B) gebildet, auf dem Luft entweder zur Gesichtsmaske 110 oder umgekehrt in die Umgebung (Pfeil B) strömt.

Die Filtereinrichtung 60 ist in der zweiten Atemleitung 30 zwischen der Sensoreinrichtung 40 und der Gesichtsmaske 110 angeordnet. Die Filtereinrichtung 60 umfasst einen vorzugsweise austauschbaren Hepa-Filter 61. Der Hepa-Filter 61 sitzt vorzugsweise direkt auf der Gesichtsmaske 110, so dass vorteilhafterweise das Totvolumen der Beatmungsvorrichtung 100 minimiert wird.

Zur Befeuchtung, Temperierung und Einstellung des Sauerstoffgehalts der Atemluft ist die Atemluftkonditionierungseinrichtung 70 am zweiten Ende 21 der ersten Atemleitung 20 vorgesehen. Die Atemluftkonditionierungseinrichtung 70 befindet sich außerhalb des Gehäuses 101 der Beatmungsvorrichtung 100 (siehe Figur 1). In der Atemluftkonditionierungseinrichtung 70 strömt Einatmungsluft aus der Umgebung, optional angereichert mit Sauerstoff aus einer Sauerstoffflasche, über Wärmetauscher von einem Widerstandsheizelement und/oder einem Peltier-Element. Des Weiteren enthält die Atemluftkonditionierungseinrichtung 70 einen Feuchtespender, der die Einatmungsluft mit Wasserdampf und/oder wässrigen Aerosoltröpfchen belädt.

Der Drucksensor 41 und der Flusssensor 42 der Sensoreinrichtung 40 sowie der Sauerstoffsättigungssensor 120 sind über Signalleitungen (gestrichelt gezeigt) mit der Steuereinrichtung 50, insbesondere der Steuerschaltung 52, verbunden. Der Sauerstoffsättigungssensor 120 z. B. ein Pulsoximeter, ist bei Betrieb der Beatmungsvorrichtung 100 zur Erfassung der Sauerstoffsättigung im Blut mit der zu beatmenden Person gekoppelt. Die Steuereinrichtung 50 ist über eine Steuerleitung 53 mit dem Radialgebläse 10, insbesondere dessen Motorsteuerung 51, verbunden. Falls anstelle des passiven Expirationsventils ein aktiv betätigbares Dreiwegeventil der Ventileinrichtung 23 vorgesehen ist, wird dieses ebenfalls mit der Steuereinrichtung 50 angesteuert (siehe gepunktete Steuerleitung), um entsprechend dem Atemrhythmus abwechselnd in einen der zwei Zweige der ersten Atemleitung 20 zu öffnen. Optional kann auch die Atemluftkonditionierungseinrichtung 70 mit der Steuereinrichtung 50 oder mit einer externen Steuerung angesteuert werden.

Der Durchmesser der ersten und zweiten Atemleitungen beträgt z. B. 15 mm. Die Länge der zweiten Atemleitung 30 vom Radialgebläse 10 bis zur Filtereinrichtung 60 beträgt z. B. 30 mm. An der Verengung 43 hat die zweite Atemleitung 30 einen Durchmesser von z. B. 8 mm.

Abweichend von Figur 4 kann die Ventileinrichtung 23 mit dem sich in die Umgebung öffnenden Ventil auf Seiten der zweiten Atemleitung 30, z. B. an der Gesichtsmaske 110 oder zwischen der Sensoreinrichtung 40 und dem Radialgebläse 10 angeordnet sein. In diesem Fall hat die erste Atemleitung 20 vorzugsweise nur einen einzigen Zweig. Wenn die Exspiration druckkontrolliert werden soll, muss das Radialgebläse 10 jedoch maskenseitig vor dem Exspirationsausgang angeordnet sein.

Der in Figur 4 illustrierte kompakte Aufbau der Beatmungsvorrichtung 100 bietet insbesondere die folgenden Vorteile. Ein Schlauchsystem wird nicht benötigt, da das Radialgebläse 10 mit der Sensoreinrichtung 40 direkt auf das HEPA Filter 61 aufgesteckt werden kann. Ventile sind nicht zwingend erforderlich. Insbesondere ist ein externes Exspirationsventil, wie es bei Ein-Schlauch-Systemen üblich ist, nicht erforderlich. Der Ruhewiderstand der Beatmungsvorrichtung 100 liegt in der Größenordnung dessen des HEPA Filters, z. B. bei 3 mbar*s / l. Da das das pneumatische System innerhalb weniger Sekunden auf Temperaturen über 40° C temperierbar ist, kann Kondensation wirksam verhindert werden. Ein zur Systemkühlung verwendete Luftstrom kann sogar mit einer einfachen Lufthutze zur Erwärmung des angeschlossenen HEPA Filters benutzt werden. Der mechanische Aufbau der Beatmungsvorrichtung 100 mit dem Gehäuse und den Atemleitungen ist einfach, z. B. mit 3D-Druck, aus Kunststoff herstellbar.

Der Beatmungsdruck an der Auslassöffnung 12 des Radialgebläses 10 wird mit dem in Figur 5 gezeigten mehrstufigen Regelverfahren abwechselnd auf einen Einatmungsdruck oder einen Ausatmungsdruck eingestellt, wie im Folgenden erläutert wird. Das Regelverfahren umfasst vier Regelkreise I bis IV, von denen der erste (I) und der zweite (II) Regelkreis für den Betrieb der Beatmungsvorrichtung 100 notwendig sind, während der dritte (III) und der vierte (IV) Regelkreis gemäß bevorzugten Ausführungsformen vorgesehen sind. Die Regelkreise bilden eine Kaskade, in der jeder Regelkreis die Sollwerte des Regelkreises des nächstniedrigeren Levels manipuliert.

Istwerte (Führungsgrößen) der Regelkreise liefern jeweils der Drucksensor 42 für den ersten Regelkreis I, der Flusssensor 42 für den zweiten Regelkreis II, eine Ausgabe 54 der Steuerschaltung 52 für den dritten Regelkreis III und der Sauerstoffsättigungssensor 120 für den vierten Regelkreis III. In den Regelkreisen sind jeweils mit IA bis IVA bezeichnete Begrenzer vorgesehen, welche die Regelkreise innerhalb vorgegebener Grenzwerte von Zeit- und Druckparametern halten. Bei den Testschritten IB bis IVB werden vorgegebene Sollwerte mit den Istwerten verglichen, wie unten erläutert ist. Die "Δ"-Funktion berechnet bei den Schritten IC bis IVC die Änderung des jeweiligen Regler-Sollwertes auf der Basis der voreingestellten Parameter.

Der erste (innerste) Regelkreis I umfasst eine Druckregelung, mit der der Beatmungsdruck (Einatmungsdruck IPAP und Ausatmungsdruck) unter Verwendung von vorgegebenen PIP- und PEEP-Parametern (IA) als obere bzw. untere Grenzwerte eingestellt wird. Der obere Grenzwert PIP wird vom behandelnden Bediener, z. B. einem Arzt, so gewählt, dass während der Beatmung die Lunge nicht überdehnt wird, und der untere Grenzwert PEEP wird so gewählt, dass bei der Ausatmung die Alveolen nicht zusammenfallen. Der Parameter PEEP wird vorzugsweise im Bereich 5 mbar bis 15 mbar, z. B. bei 8mbar, gewählt. Der erste Regelkreis I ist ein PI-Regler, mit dem der Beatmungsdruck über die Drehzahl des Radialgebläses 10 eingestellt wird und dessen Integrationskonstanten mit den Parametern PIP und PEEP gegeben sind.

Der gesunde Mensch benötigt in Ruhe ein Atemvolumen von etwa 8 ml Luft / (kg [Körpergewicht] * min). Im Allgemeinen wird sich der Zustand des Patienten mit der Zeit ändern, z.B. eine Besserung in der Entwöhnungsphase von der Beatmung, oder eine Verschlechterung z.B. infolge fortschreitender Atelektasenbildung (Alveolenverschluss) auftreten. Um den Kollaps der Alveolen am Ende der Exspirationsphase zu vermeiden, ist das Aufrechterhalten des Minimaldruckes (PEEP, positive end-exspiratory pressure) vorgesehen. Entsprechend muss die Beatmungsvorrichtung 100 nur variierende positive Drucke generieren.

Die nächste höhere Stufe umfasst mit dem zweiten Regelkreis II eine Zeitsteuerung, deren Sollwerte für Frequenz (f) der Einatmung und Tastverhältnis von Ein- und Ausatmung (I:E) innerhalb von physiologisch sinnvollen Grenzen (parametrierbar) eingestellt sind. Zusammen mit der unterlagerten Druckregelung ist bereits die BIPAP Funktionalität implementiert. Die Frequenz wird z. B. im Bereich von 25 min$^{-1}$ bis 12 min$^{-1}$ gewählt. Das Tastverhältnis wird z. B. im Bereich von 30 % (bei niedrigen Frequenzen) bis 50 % (bei höheren Frequenzen) gewählt.

Auf dem zweithöchsten Level wird mit dem dritten Regelkreis III eine Regelung des Tidal- bzw. Minutenvolumens (TV, MV) implementiert, um auf Änderungen der Steifigkeit der Lunge und den Atemwegswiderstand innerhalb vorgegebener Grenzen (parametrierbar) reagieren zu können. Führungsgröße ist das durch zeitliche Integration des gemessenen Flusses während Inspirations- und Exspirationsphasen errechnete und von der Steuerschaltung 52 am Ausgang 54 bereitgestellte Tidalvolumen. Der Sollwert für den dritten Regelkreis III wird durch den Parameter Minutenvolumen vorgegeben. Der Ausgang des dritten Regelkreises III modifiziert die Sollwertvorgaben der Druckregelung des ersten Regelkreises I und der Zeitsteuerung des zweiten Regelkreises II.

Auf dem höchsten Level wird mit dem vierten Regelkreis IV der Sollwert des Minutenvolumens durch einen i-Regler vorgegeben, der die Sauerstoffsättigung als Führungsgröße hat und mit seinem lokalen Sollwert vergleicht. Hiermit wird der gesamte Regelkreis geschlossen. Zu niedrige Sauerstoffsättigung z.B. führt zur allmählichen Erhöhung der Minutenvolumen-Vorgabe, und diese zur Erhöhung der Sollwerte für PIP, Atemfrequenz, Inspirations-/Exspirationsverhältnis und PEEP.

Die Grenzen der Bereiche aller Sollwerte sind parametriert. Damit bleiben die Regelungen der einzelnen Regelkreise bei Nichtvorhandensein einer Führungsgröße oder Stellgröße einer höheren Ebene automatisch im Bereich dessen, was durch ihre lokale Parametrierung vorgegeben ist. Ein Ausfall des Sauerstoffsättigungssensors 120 würde z.B. zum Einfrieren des Sollwertes des Minutenvolumens führen und bei aktivierter Regelung im vierten Regelkreis IV zu einer Warnung führen. Sind gewünschte Minutenvolumen aufgrund z.B. zu hoher Compliance nicht innerhalb der vorgegebenen Grenzen der Zeitsteuerung bzw. Druckregelung realisierbar, bleibt das System automatisch in diesen Grenzen und gibt eine Warnung aus.

Bei Betrieb der Beatmungsvorrichtung 100 ist vorzugsweise mindestens eine der folgenden Sicherheitsmaßnahmen vorgesehen. Bei Stromausfall geht das Verhalten der Beatmungsvorrichtung 100 in das einer FFP3-Maske über. Die Maximaldrehzahl des Radialgebläses 10 und damit der erreichbare Systemdruck sind durch das Lüfterdesign, die Betriebsspannung und den Durchmesser des Lüfterrades unveränderbar festgelegt. Ein Ausfall der Motorsteuerung 51 kann nicht zum Durchlaufen des Motors führen, und ein Durchgehen wie bei DC Motoren ist nicht möglich. Es gibt keine blockierenden Ventile, Ein und Ausatemwege sind immer voll offen und werden nur dynamisch bedruckt. Die Verwendbarkeit nicht belüfteter Masken ist damit ohne Einschränkung gegeben. Kondenswasserbildung ist nach einer kurzen Aufwärmphase unter anderem aufgrund der fehlenden Schlauchleitungen per Design unterbunden.

Um bei der NIV-Beatmung eventuelle Verluste durch Leckagen der Gesichtsmaske abschätzen zu können, gibt es mindestens zwei Möglichkeiten. Erstens ergibt sich durch eine Messung und Extrapolation der Fluss und Druckdaten am Ende der Inspirationsphase eine Abschätzung der durch Leckagen der Maske versursachte Fluss bei gleichbleibendem Druck. Zweitens kann eine bidirektionale Messung des Flusses vor dem Radialgebläse vorgesehen sein.

Zusammenfassend hat die Beatmungsvorrichtung 100 vorteilhafterweise mindestens eines der folgenden Merkmale. Mit der Beatmungsvorrichtung 100 wird sichergestellt, dass die beatmete Person genügend Sauerstoff erhält (Sauerstoffsättigung typischerweise > 90%), und dass genügend $CO_2$ entfernt wird, um seine Lebensfunktionen zu erhalten. Das System ist dafür ausgelegt, die Führungsgröße Sauerstoffsättigung ($SpO_2$) zu erfassen. Es kann zusätzlichen Sauerstoff eingespeist werden. Die Beatmungsvorrichtung 100 kann innerhalb physiologisch sinnvoller Grenzen autark Änderungen der Lungenkondition der beatmeten Person durch Änderung des Tidal- bzw. Minutenvolumens kompensieren. Die Beatmungsvorrichtung 100 kann durch akustische, optische und/oder digitale Alarme dem Klinikpersonal signalisieren können, wann die Destabilisierung der Therapie droht.

Die in der vorstehenden Beschreibung, den Zeichnungen und den Ansprüchen offenbarten Merkmale der Erfindung können sowohl einzeln als auch in Kombination oder Unterkombination für die Verwirklichung der Erfindung in ihren verschiedenen Ausgestaltungen von Bedeutung sein.

**Patentansprüche**

1.   Beatmungsvorrichtung (100), die zur nicht-invasiven Beatmung einer Person eingerichtet ist, umfassend

  - ein Radialgebläse (10) mit einer Einlassöffnung (11) und einer Auslassöffnung (12), wobei das Radialgebläse (10) zur Aufnahme von Beatmungsluft an der Einlassöffnung (11) und zur Bereitstellung der Beatmungsluft mit einem einstellbaren Beatmungsdruck an der Auslassöffnung (12) eingerichtet ist,
  - eine erste Atemleitung (20) mit einem ersten Ende (21), das mit der Einlassöffnung (11) des Radialgebläses (10) verbunden ist, und mit einem zweiten Ende (22), das zur Aufnahme von Einatemluft angeordnet ist,
  - eine zweite Atemleitung (30) mit einem ersten Ende (31), das mit der Auslassöffnung (12) des Radialgebläses (10) verbunden ist, und mit einem zweiten Ende (32), das für eine Ankopplung einer Atemmaske (110) eingerichtet ist, wobei die zweite Atemleitung (30) mit einer Sensoreinrichtung (40) ausgestattet ist, mit der mindestens ein Strömungsparameter in der zweiten Atemleitung (30) messbar ist, und
  - eine Steuereinrichtung (50), die zur Steuerung des Radialgebläses (10) in Abhängigkeit von mindestens einem Ausgangssignal der Sensoreinrichtung (40) eingerichtet ist, wobei
  - das Radialgebläse (10) und die zweite Atemleitung (30) einen durchgehenden bidirektionalen Strömungsweg bilden, wobei der einstellbare Beatmungsdruck an der Auslassöffnung (12) des Radialgebläses (10) einen Einatmungsdruck oder einen Ausatmungsdruck umfasst, der gleich dem oder geringer als der Einatmungsdruck ist,
  - die zweite Atemleitung (30) mit einer Filtereinrichtung (60) ausgestattet ist, die zur Luftströmungsfilterung angeordnet ist, und
  - die Steuereinrichtung (50) zur Steuerung des Radialgebläses (10) mit einem ersten Regelkreis (I) eingerichtet ist, mit dem der Beatmungsdruck an der Auslassöffnung (12) des Radialgebläses (10) einstellbar ist, wobei ein Istwert des ersten Regelkreises von einem Drucksensor (42) geliefert wird,

  **dadurch gekennzeichnet, dass**

  - die Steuereinrichtung (50) zur Steuerung des Radialgebläses (10) mit einem zweiten Regelkreis (II) eingerichtet ist, mit dem mindestens eines einstellbar ist von einer Frequenz von Einatmungsphasen, in denen der Einatmungsdruck an der Auslassöffnung (12) des Radialgebläses (10) gebildet ist, und einem Tastverhältnis der Dauer der Einatmungsphasen relativ zu der Dauer von Ausatmungsphasen, in denen der Ausatmungsdruck an der Auslassöffnung (12) des Radialgebläses (10) gebildet ist, wobei ein Istwert des zweiten Regelkreises von einem Flusssensor (42) geliefert wird.

2.   Beatmungsvorrichtung gemäß Anspruch 1, bei der

  - der Strömungsweg zwischen dem zweiten Ende (32) der zweiten Atemleitung (30) und der Einlassöffnung (11) des Radialgebläses (10) frei von Ventilen ist.

3.   Beatmungsvorrichtung gemäß einem der vorhergehenden Ansprüche, bei der

  - die erste Atemleitung (20) zwischen der Einlassöffnung (11) des Radialgebläses (10) und dem zweiten Ende (22) der ersten Atemleitung (20) eine Ventileinrichtung (23), insbesondere ein passives Auslassventil, enthält, die als ein Exspirationsausgang zum Auslass von Ausatemluft angeordnet ist.

4.   Beatmungsvorrichtung gemäß einem der vorhergehenden Ansprüche, bei der

  - das zweite Ende (22) der ersten Atemleitung (20) sich in eine Umgebung der Beatmungsvorrichtung (100) öffnet und/oder für eine Ankopplung an ein Atemluftreservoir (300) eingerichtet ist.

5.   Beatmungsvorrichtung gemäß einem der vorhergehenden Ansprüche, die umfasst

- eine Atemluftkonditionierungseinrichtung (70), die mit dem zweiten Ende der ersten Atemleitung (20) gekoppelt ist.

6. Beatmungsvorrichtung gemäß Anspruch 5, bei der

- die Atemluftkonditionierungseinrichtung (70) zur Einstellung von mindestens einem der Parameter Feuchte, Temperatur und Sauerstoffgehalt der Einatemluft eingerichtet ist.

7. Beatmungsvorrichtung gemäß Anspruch 6, bei der

- die Atemluftkonditionierungseinrichtung (70) eine Temperierungseinrichtung (71) umfasst, die zur Temperierung der Beatmungsvorrichtung (100), insbesondere der zweiten Atemleitung (30), eingerichtet ist.

8. Beatmungsvorrichtung gemäß einem der vorhergehenden Ansprüche, mit mindestens einem der Merkmale

- die Filtereinrichtung (60) umfasst einen HEPA Filter, und
- die Filtereinrichtung (60) weist eine Steckverbindung mit dem Radialgebläse (10) und/oder der zweiten Atemleitung (30) auf.

9. Beatmungsvorrichtung gemäß einem der vorhergehenden Ansprüche, mit mindestens einem der Merkmale

- die Steuereinrichtung (50) ist mit einer Schnittstelle zur Aufnahme eines Ausgangssignals eines Sauerstoffsättigungssensors ausgestattet,
- die Steuereinrichtung (50) ist unmittelbar an das Radialgebläse (10) angrenzend angeordnet, und
- die Steuereinrichtung (50) ist zur Ausgabe eines Alarmsignals bei Feststellung einer Fehlfunktion der Beatmungsvorrichtung und/oder eines bedenklichen Zustands der beatmeten Person eingerichtet.

10. Beatmungsvorrichtung gemäß einem der vorhergehenden Ansprüche, bei der

- die Steuereinrichtung (50) zur Steuerung des Radialgebläses (10) mit einem dritten Regelkreis (III) eingerichtet ist, mit dem Sollgrößen des ersten und/oder des zweiten Regelkreises (I, II) derart einstellbar sind, dass mindestens einer der Parameter Tidalvolumen und Minutenvolumen der Beatmung an vorbestimmte Volumen-Referenzgrößen des Tidalvolumens oder des Minutenvolumens angepasst werden.

11. Beatmungsvorrichtung gemäß Anspruch 10, bei der

- die Steuereinrichtung (50) zur Steuerung des Radialgebläses (10) in Abhängigkeit von einem Ausgangssignal eines Sauerstoffsättigungssensors mit einem vierten Regelkreis (IV) eingerichtet ist, mit dem eine Sollgröße des dritten Regelkreises (III) derart einstellbar ist, dass eine Sauerstoffsättigung im Blut der beatmeten Person an eine vorbestimmte Sauerstoffsättigungs-Referenzgröße angepasst wird.

## Claims

1. A ventilator device (100) configured for non-invasive ventilation of a person, comprising

- a radial blower (10) having an inlet opening (11) and an outlet opening (12), wherein the radial blower (10) is configured to receive ventilation air at the inlet opening (11) and to provide the ventilation air with an adjustable ventilation pressure at the outlet opening (12),
- a first ventilation duct (20) having a first end (21), connected to the inlet opening (11) of the radial blower (10), and having a second end (22), arranged to receive inhalation air,
- a second ventilation duct (30) having a first end (31), connected to the outlet opening (12) of the radial blower (10), and having a second end (32), configured for coupling to a breathing mask (110), wherein the second ventilation duct (30) is equipped with a sensor device (40), by which at least one flow parameter in the second ventilation duct (30) can be measured, and
- a control device (50) configured to control the radial blower (10) as a function of at least one output signal from the sensor device (40), wherein
- the radial blower (10) and the second ventilation duct (30) form a continuous bidirectional flow path, wherein the adjustable ventilation pressure at the outlet opening (12) of the radial blower (10) comprises an inhalation pressure or an exhalation pressure, equal to or less than the inhalation pressure,
- the second ventilation duct (30) is equipped with a filter device (60), arranged for air flow filtering, and
- the control device (50) is, for controlling the radial blower (10), configured with a first control loop (I), by which the ventilation pressure at the outlet opening (12) of the radial blower (10) can be adjusted,
**characterized in that**

- the control device (50) is, for controlling the radial blower (10), configured with a second control loop (II), by which at least one of a frequency of inhalation phases, in which the inhalation pressure is formed at the outlet opening (12) of the radial blower (10), and a duty cycle of the duration of the inhalation phases relative to the duration of exhalation phases in which the exhalation pressure is formed at the outlet opening (12) of the radial blower (10), can be set, wherein an actual value of the second control loop is supplied by a flow sensor (42).

2. The ventilator device (100) according to claim 1, wherein

   - the flow path between the second end (32) of the second ventilation duct (30) and the inlet opening (11) of the radial blower (10) is free of valves.

3. The ventilator device (100) according to any one of the preceding claims, wherein

   - the first ventilation duct (20) between the inlet opening (11) of the radial blower (10) and the second end (22) of the first ventilation duct (20) comprises a valve device (23), in particular a passive outlet valve, arranged as an expiratory outlet for discharging exhalation air.

4. The ventilator device (100) according to any one of the preceding claims, wherein

   - the second end (22) of the first ventilation duct (20) opens into an environment of the ventilator device (100) and/or is adapted for coupling to a breathing air reservoir (300).

5. The ventilator device (100) according to any one of the preceding claims, wherein

   - a breathing air conditioning device (70) coupled to the second end of the first ventilation duct (20).

6. The ventilator device (100) according to claim 5, wherein

   - the breathing air conditioning device (70) is arranged to adjust at least one of the parameters humidity, temperature, and oxygen content of the inhalation air.

7. The ventilator device (100) according to claim 6, wherein

   - the breathing air conditioning device (70) comprises a tempering device (71) configured to temper the ventilator device (100), in particular the second ventilation duct (30).

8. The ventilator device (100) according to any one of the preceding claims, having at least one of the features

   - the filter device (60) comprises a HEPA filter, and
   - the filter device (60) comprises a plug-in connection with the radial blower (10) and/or the second ventilation duct (30).

9. The ventilator device (100) according to any one of the preceding claims, having at least one of the features

   - the control device (50) is equipped with an interface for receiving an output signal from a oxygen saturation sensor,
   - the control device (50) is arranged directly adjacent to the radial blower (10), and
   - the control device (50) is configured to output an alarm signal when a malfunction of the ventilator device and/or a critical condition of the ventilated person is detected.

10. The ventilator device (100) according to any one of the preceding claims, wherein

    - the control device (50) is, for controlling the radial blower (10), configured with a third control loop (III), by which setpoint variables of the first and/or the second control loop (I, II) can be set such that at least one of the parameters tidal volume and minute volume of the ventilation are adapted to predetermined volume reference variables of the tidal volume or the minute volume.

11. The ventilator device (100) according to claim 10, wherein

    - the control device (50) is, for controlling the radial blower (10), configured, as a function of an output signal of an oxygen saturation sensor, with a fourth control loop (IV), by which a setpoint variable of the third control loop (III) can be set such that an oxygen saturation in the blood of the ventilated person is adapted to a predetermined oxygen saturation reference variable.

**Revendications**

1. Dispositif respiratoire (100), qui est mis au point pour ventiler de manière non invasive une personne, comprenant

- une soufflante radiale (10) avec un orifice d'entrée (11) et un orifice de sortie (12), dans lequel la soufflante radiale (10) est mise au point pour recevoir de l'air de ventilation sur l'orifice d'entrée (11) et pour fournir l'air de ventilation à une pression de ventilation réglable sur l'orifice de sortie (12),
- une première conduite de respiration (20) avec une première extrémité (21), qui est reliée à l'orifice d'entrée (11) de la soufflante radiale (10), et avec une seconde extrémité (22), qui est disposée pour recevoir l'air inspiré,
- une seconde conduite de respiration (30) avec une première extrémité (31), qui est reliée à l'orifice de sortie (12) de la soufflante radiale (10), et avec une seconde extrémité (32), qui est mise au point pour un couplage d'un masque respiratoire (110), dans lequel la seconde conduite de respiration (30) est équipée d'un système capteur (40) avec lequel l'au moins un paramètre d'écoulement peut être mesuré dans la seconde conduite de respiration (30), et
- un système de commande (50), qui est mis au point pour commander la soufflante radiale (10) en fonction d'au moins un signal de sortie du système capteur (40), dans lequel
- la soufflante radiale (10) et la seconde conduite de respiration (30) forment un trajet d'écoulement bidirectionnel continu, dans lequel la pression de ventilation réglable sur l'orifice de sortie (12) de la soufflante radiale (10) comprend une pression d'inspiration ou une pression d'expiration qui est inférieure ou égale à la pression d'inspiration,
- la seconde conduite de respiration (30) est équipée d'un système de filtrage (60), qui est disposé pour filtrer le flux d'air, et
- le système de commande (50) est mis au point pour commander la soufflante radiale (10) avec un premier circuit de régulation (I), avec lequel la pression de ventilation sur l'orifice de sortie (12) de la soufflante radiale (10) peut être réglée, dans lequel une valeur réelle du premier circuit de régulation est fournie par un capteur de pression (42),
**caractérisé en ce que**
- le système de commande (50) est mis au point pour commander la soufflante radiale (10) avec un deuxième circuit de régulation (II), avec lequel au moins un élément d'une fréquence de phases d'inspiration, au cours de laquelle la pression d'inspiration est formée sur l'orifice de sortie (12) de la soufflante radiale (10), et d'un rapport cyclique de la durée des phases d'inspiration par rapport à la durée des phases d'expiration, au cours desquelles la pression d'expiration est formée sur l'orifice de sortie (12) de la soufflante radiale (10), peut être réglé, dans lequel une valeur réelle du deuxième circuit de régulation est fournie par un capteur de débit (42).

2. Dispositif respiratoire selon la revendication 1, dans lequel

- le chemin d'écoulement entre la seconde extrémité (32) de la seconde conduite de respiration (30) et l'orifice d'entrée (11) de la soufflante radiale (10) est sans vannes.

3. Dispositif respiratoire selon l'une quelconque des revendications précédentes, où

- la première conduite de respiration (20) contient entre l'orifice d'entrée (11) de la soufflante radiale (10) et la seconde extrémité (22) de la première conduite de respiration (20) un système de valve (23), notamment une valve d'échappement passive, qui est disposée comme une sortie d'expiration pour la sortie de l'air expiré.

4. Dispositif respiratoire selon l'une quelconque des revendications précédentes, où

- la seconde extrémité (22) de la première conduite de respiration (20) s'ouvre dans un environnement du dispositif respiratoire (100) et/ou est configurée pour être couplée à un réservoir d'air de respiration (300).

5. Dispositif respiratoire selon l'une quelconque des revendications précédentes, qui comprend

- un système de conditionnement d'air de respiration (70) qui est couplé à la seconde extrémité de la première conduite de respiration (20).

6. Dispositif respiratoire selon la revendication 5, où

- le système de conditionnement d'air de respiration (70) est configuré pour régler au moins un des paramètres humidité, température et teneur en oxygène de l'air inspiré.

7. Dispositif respiratoire selon la revendication 6, où

- le système de conditionnement d'air de respiration (70) comprend un système de thermorégulation (71), qui est mis au point pour thermoréguler le dispositif respiratoire (100), en particulier la seconde conduite de respiration (30).

8. Dispositif respiratoire selon l'une quelconque des revendications précédentes, avec au moins une des caractéristiques

- le système filtrant (60) comprend un filtre HEPA, et
- le système de filtrage (60) présente une connexion enfichable avec la soufflante radiale (10) et/ou la seconde conduite de respiration (30).

9. Dispositif respiratoire selon l'une quelconque des revendications précédentes, avec au moins une des caractéristiques

- le système de commande (50) est équipé d'une interface de réception d'un signal de sortie d'un capteur de saturation en oxygène,
- le système de commande (50) est disposé directement de manière adjacente à la soufflante radiale (10), et
- le système de commande (50) est mis au point pour émettre un signal d'alarme en cas de détection d'un dysfonctionnement du dispositif respiratoire et/ou d'un état préoccupant de la personne ventilée.

10. Dispositif respiratoire selon l'une quelconque des revendications précédentes, où

- le système de commande (50) est mis au point pour commander la soufflante radiale (10) avec un troisième circuit de régulation (III), avec lequel des grandeurs de consigne du premier et/ou du deuxième circuit de régulation (I, II) peuvent être réglées de telle manière qu'au moins un des paramètres volume courant et débit-volume de la ventilation est adapté à des valeurs de référence de volume prédéfinies du volume courant ou du débit-volume.

11. Dispositif respiratoire selon la revendication 10, où

- le système de commande (50) est mis au point pour commander la soufflante radiale (10) en fonction d'un signal de sortie d'un capteur de saturation en oxygène avec un quatrième circuit de régulation (IV), avec lequel une grandeur de consigne du troisième circuit de régulation (III) peut être réglée de telle manière qu'une saturation en oxygène du sang de la personne ventilée est adaptée à une valeur de référence de saturation en oxygène prédéfinie.

FIG. 1

FIG. 2

FIG. 3

FIG. 4

EP 4 181 992 B1

120

IV

IVB

$<> SpO2_c\ ?$

IVA

$(TV\ |\ MV)_s => (TV\ |\ MV)_s + \Delta(SpO2,...)$

IVC

54

III

IIIB

$<> (TV\ |\ MV)_c\ ?$

IIIA

$(f\ |\ (I:E))_s => (f\ |\ (I:E))_s + \Delta((TV\ |\ MV),...)$

IIIC

41

II

IIB

$<> (f\ |\ (I:E))_c\ ?$

IIA

$(PEEP\ |\ IPAP)_s => (PEEP\ |\ IPAP)_s + \Delta((f\ |\ (I:E),...)$

IIC

42

I

IB

$<> PEEP\ |\ IPAP_c\ ?$

IA

IC

$(PEEP\ |\ IPAP)_s => (PEEP\ |\ IPAP)_s + \Delta((PEEP\ |\ IPAP),...)$

FIG. 5

**IN DER BESCHREIBUNG AUFGEFÜHRTE DOKUMENTE**

**In der Beschreibung aufgeführte Patentdokumente**

- DE 102008047026 B4
- WO 2009112076 A1
- DE 102008005558 A1
- DE 102018003027 A1
- DE 20016769 U1

- US 2019269871 A1
- US 2018064894 A1
- JP 2018531716 A
- WO 2018204985 A1

**In der Beschreibung aufgeführte Nicht-Patentliteratur**

- **B. C. GALBIATI et al.** *arXiv:2003.10405*, 2020